# EUROPEAN PATENT SPECIFICATION

(11) **EP 1 835 911 B1**
(45) Date of publication and mention of the grant of the patent: **29.08.2012**
(21) Application number: 05854564.1
(22) Date of filing: 15.12.2005
(51) Int. Cl.: A61K 31/407, A61K 31/167, A61K 9/08, A61P 25/06, A61P 29/00

(54) **THERAPEUTIC COMPOSITIONS FOR INTRANASAL ADMINISTRATION OF KETOROLAC**
THERAPEUTISCHE ZUSAMMENSETZUNGEN FÜR DIE INTRANASALE VERABREICHUNG VON KETOROLAC
COMPOSITIONS THERAPEUTIQUES SERVANT A ADMINISTRER KETOROLAC PAR VOIE NASALE

(30) Priority: 23.12.2004 US 639022 P
(43) Date of publication of application: 26.09.2007
(73) Proprietor: ROXRO Pharma, Inc., Menlo Park, CA 94025-3444 (US)
(72) Inventor: WHITING, Roger, Los Altos, California 94022 (US); THIRUCOTE, Ramachandran, Redwood Shores, California 94065 (US)
(74) Representative: Wytenburg, Wilhelmus Johannes
(86) International application number: PCT/US2005/045883
(87) International publication number: WO 2006/071601

(56) References cited:
- GB-A- 2 315 673
- US-A- 6 090 368
- US-A1- 2003 022 894
- US-B1- 6 333 044
- REUBEN S S ET AL: "AN EVALUATION OF THE ANALGESIC EFFICACY OF INTRAVENOUS REGIONAL ANESTHESIA WITH LIDOCAINE AND KETOROLAC USING A FOREARM VERSUS UPPER ARM TOURNIQUET" ANESTHESIA AND ANALGESIA, WILLIAMS AND WILKINS, BALTIMORE, MD, US, vol. 95, no. 2, 2002, pages 457-460, XP008024919 ISSN: 0003-2999

## Description

### Field of the Invention

This invention relates to therapeutic compositions with analgesic and anti-inflammatory activity, suitable for intranasal administration, which include ketorolac or its pharmaceutically acceptable salts as the active ingredient and a local anesthetic to reduce the sensation of stinging and to improve efficacy. This invention also relates to a therapeutic method that provides for the nasal administration of the composition to a subject to treat pain, wherein the subject has a reduced sensation of stinging and the efficacy is improved compared to a known composition.

### Background of the Invention

Ketorolac or 5-benzoyl-2,3-dihydro-1H-pyrrolizine-1-carboxylic acid has the following formula (I): 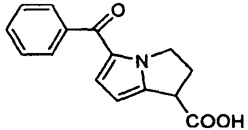 It has been known for several years (U.S. Patent No. 4,089,969) and is used in human therapy as an analgesic and an anti-inflammatory as the tromethamine salt.

Both the racemic form and each of the dextro and levo isomers of this compound are known. Many pharmaceutically acceptable salts, the most commonly used of which is the tromethamine (2-amino-2-hydroxymethyl-1,3-propanediol) salt, are also known. Hereinafter, the name ketorolac shall encompass individually or collectively the racemic mixture or either optically active compound and shall encompass the free acid as well as the tromethamine salt or any other pharmaceutically acceptable salt of any one of the foregoing.

Ample literature is available on ketorolac (for instance, "Ketorolac - A review of its pharmacodynamic and pharmacokinetic properties and its therapeutic potential", Drugs 39(1): 86-109, 1990). It is described as a drug with considerably higher analgesic activity than many other non-steroidal anti-inflammatory drugs. Most significantly, it has analgesic activity comparable to that of the opiates, such as morphine, without the well-known side effects of the latter.

It's known that ketorolac can be formulated as a nasally administratable composition. See U.S. Patent 6,333,044 to Recordati. However, it is found that in some subjects there is an adverse local reaction in the nasal passages that results in the sensation of stinging or irritation. While the nasal composition of ketorolac has many advantages discussed in the Recordati patent, to help patient acceptance, any stinging sensation should be minimized while at the same time not adversely affecting the pharmacokinetic profile of the nasal formulation.

A number of substances are known as local anesthetics and are applied topically in various situations to deaden the sensation of pain. These local anesthetics are generally known not to be active in solution and thus are generally applied as suspensions or in some topical formulation wherein the active anesthetic is not dissolved. Surprisingly, it has now been found that a solution of a local anesthetic can be used with ketorolac to minimize the stinging sensation in certain people who experience stinging when ketorolac is nasally administered.

US Patent Application Publication No. 2003/0022894 Al a describes a composition that is a combination of a cGMP PDE V inhibitor in combination with a local anesthetic for nasal administration. As discussed in that publication, there are certain problems in attempting to administer a PDE V inhibitor (e.g., Viagra®). The PDE V inhibitors are vasodilators, and nasal administration leads to multiple dilation of the vessels of the nasal mucosa, and this, it is claimed, results in itching, stinging, eye watering, or other irritation in certain patients. The amount of the local anesthetic used in that dosage form is generally distinctly less than that necessary to obtain topical anesthesia. Specifically, in paragraph [0072] of U.S. Patent Publication 2003/0022894 Al, it is taught that "the compositions... contain the local anaesthetic(s) in lower concentrations than the standard amount in commercially available topical preparations for surface anaesthesia, namely in a concentration of less than 4% (m/v)." Contrary to the teaching of that US patent publication, we have found that the stinging due to the ketorolac administration (ketorolac not being a vasodilator) can be decreased by using an amount more than 4% w/v (i.e., weight volume, which is interchangeable with mass volume - m/v and is defined as the mass of a substance in grams ("g") divided by the volume of the solution in liters ("L") times 100%: % g/L) in the composition as a solution to reduce the stinging problem as experienced by certain members of the population.

UK patent application GB 2315673 A is drawn to the treatment of migraine by administering a local anesthetic and a 5-HT1D agonist intranasally. This is based on the hypothesis that the absorption of the 5-HT1D agonist will be enhanced by the presence of the local anesthetic, which is thought to have a vasodilatative effect. It is claimed that increased absorption should lead to faster distribution and onset of action of the 5HT1D agonist. No data is provided to support this hypothesis, and no mention is made regarding the time that the maximum concentration of the 5HT1D is reached. Surprisingly, it has now been found that in preferred formulations of the invention the time (Tmax) for ketorolac to reach its maximum, or peak, concentration (Cmax) is shortened thus providing better pain relief.

### Summary of the Invention

One aspect of this invention is a composition for nasal administration that comprises an effective amount of the compound 5-benzoyl-2,3-dihydro-1H-pyrrolizine-1-carboxylic acid, of the formula (I) 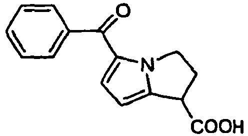 an optically active form thereof, the racemic mixture,or a pharmaceutically acceptable salt thereof, in combination with a pharmaceutically acceptable diluent and 4% - 10% w/v of a local anesthetic, e.g., lidocaine hydrochloride. Preferably the compound is ketorolac tromethamine present at a level of about 2.5 - 22.5% (w/v), the anesthetic is present at a level of 5% to 10% w/v, and the composition is a sprayable aqueous solution.

Another aspect of this invention is a composition for spraying into a human subject's nasal passage that comprises
(a) water;
(b) the compound 5-benzoyl-2,3-dihydro-1H-pyrolizine-1-carboxylic acid, an optically active form thereof, a racemic mixture, or a pharmaceutically acceptable salt (e.g., tromethamine) wherein the compound is dissolved in water at a level sufficient to be absorbed by the subject to treat pain or inflammation;
(c) a local anesthetic (e.g., lidocaine hydrochloride) dissolved in the water at a level sufficient to reduce any stinging sensation caused by nasally administering the compound alone to the subject; and
(d) optionally other pharmaceutically acceptable excipients.

Another aspect of this invention is the composition of this invention for use in a method for treating pain or inflammation in a subject in need of such treatment, which comprises the intranasal administration

Another aspect of the invention is the composition of the invention in a vessel equipped with a device for spraying the composition into a patient's nasal passage.

Another aspect of the invention is the use of the compound 5-benzoyl-2,3-dihydro-1-H-pyrrolizine-1-carboxylic acid, an optically active form thereof, the racemic mixture, or a pharmaceutically-acceptable salt thereof, in combination with a local anesthetic, to prepare a composition for nasal administration to a subject for the treatment of pain or inflammation.

An important characteristic of all aspects of the invention is that the anesthetic is chosen to be present at a level that does not adversely affect the plasma pharmacokinetics (PK) of ketorolac.

### Drawings

Figure 1 shows the mean linear and semi-logarithmic plasma ketorolac concentration profiles over 24 hours.

Figure 2 shows the mean linear and semi-logarithmic lidocaine concentration over 24 hours.

### Detailed Description of the Invention

We have now found that it is possible to prepare analgesic/anti-inflammatory formulations containing ketorolac as an active ingredient, for the treatment of pain and/or inflammation in a human subject The formulations are suitable for intranasal administration of a therapeutically effective amount to a subject so that ketorolac so administered is rapidly and thoroughly absorbed, giving therapeutic effects equivalent to or better than those obtained by the previously described intranasal formulation with reduced stinging experienced by some subjects. The formulation is designed to employ an amount of the anesthetic that reduces the sensation of stinging in those patients that might otherwise experience such a sensation upon nasal administration, while not resulting in a sensation of numbness. Importantly, it appears that the PK of ketorolac are not adversely affected by the presence of the anesthetic. That is, the plasma ketorolac concentration vs. time is not significantly modified in an adverse manner by the presence of the anesthetic. Surprisingly, the presence of the anesthetic, e.g. lidocaine hydrochloride at the preferred level of 5-6% w/v, actually decreases the Tmax for ketorolac to reach its Cmax in a subject's plasma. This provides an unexpected advantage over a nasal formulation of ketorolac without lidocaine hydrochloride, in that a faster time to Cmax generally provides a subject with better pain relief.

The intranasal formulations of the invention contain ketorolac concentrations ranging from 2.5 to 22.5%, for example 5 % to 20%, preferably about 15% w/v based on the final formulation. Alternatively this can be expressed as 25-225 milligrams (mg) per milliliter (mL), preferably 50-200 mg/mL, and most preferably 150 mg/mL. Of course, the selection of the particular excipients depends on the desired formulation dosage form, i.e., on whether a solution to be used in drops or as a spray (aerosol) is desired or whether a suspension, ointment or gel to be applied directly to the nasal cavity is desired. In any case, the invention enables the preparation of single-dose or multi-dose dosage forms, which ensure application of an optimum quantity of drug. Generally, a subject is administered about 25 to 200 microliters of the sprayable aqueous formulation of this invention, preferably 50 to 100 microliters, in one or both nostrils. For example, 100µL of a 15% (pH 7.2) w/v solution of ketorolac into each nostril, would result in up to 30 mg of ketorolac in the subject's plasma. While the composition is usually administered four times a day, under certain circumstances it may be administered less frequently if appropriate. Intranasal administration of a composition according to the invention in amounts ranging between 0.5 milligrams (mg)/kilograms (kg)/day and 4 mg/kg/day will generate plasma levels of ketorolac within the range of 0.3-5 mg/L of plasma, which is generally efficacious in treating moderate to severe pain, whether of a pathological or neuropathic origin, such as trauma-inflicted pain, post-operative pain, migraine, and the like. In general, a subject that is 18 to 65 years old could receive up to 120 mg per day of ketorolac by intranasal administration of 100 microliters per nostril of a 15% w/v ketorolac solution 4 times per day. A subject that is an adolescent or is older than 65 could receive 60 mg per day by intranasal administration of 50 microliters per nostril of a 15% w/v ketorolac formulation 4 times a day. Children 12 and under would receive appropriately less.

The preferred diluent for the formulations according to the invention is water, and other excipients may be added if desired. The preferred 15% w/v ketorolac formulation is hypertonic, i.e., it exhibits an osmotic pressure that is greater than that of biological fluids. In cases where the concentration of ketorolac is lower, it may be useful to add an isotonicity agent selected among those commonly used in pharmaceutics. Substances used for this purpose are, for instance, sodium chloride and glucose. The quantity of isotonicity agent will depend on the desired osmotic pressure of the formulation (taking into account the osmotic effect of the active ingredient). Generally this will be in the range of about 150 to about 850 milliOsmoles (mOsm).

Should a suspension or gel be desired instead of a solution, appropriate oily or gel vehicles may be used or one or more polymeric materials may be included, which desirably should be capable of conferring bioadhesive characteristics to the vehicle.

Several polymers are used in pharmaceutics for the preparation of a gel; the following can be mentioned as nonlimiting examples: hydroxypropyl cellulose (KLUCEL®), hydroxypropyl methyl cellulose (METHOCEL®), hydroxyethyl cellulose (NATROSOL®), sodium carboxymethyl cellulose (BLANOSE®), acrylic polymers (CARBOPOL®, POLYCARBOPHIL®), gum xanthan, gum tragacanth, alginates and agar-agar. The parenthetical phase provides a tradename of a product available on the open market.

Some of them, such as sodium carboxymethyl cellulose (often abbreviated as sodium CMC) and acrylic polymers, have marked bioadhesive properties and are preferred ifbioadhesiveness is desired.

Other formulations suitable for intranasal administration ofketorolac can be obtained by adding to the aqueous vehicle polymers capable of changing the rheologic behavior of the composition in relation to the temperature.

These polymers make it possible to obtain low viscosity solutions at room temperature, which can be applied for instance by nasal spray and which increase in viscosity at body temperature, yielding a viscous fluid which increases the likelihood of a longer contact with the nasal mucous membrane. Polymers of this class include without limitation polyoxyethylene-polyoxypropylene block copolymers (POLOXAMER®). Polyoxyethylene is often abbreviated as POE, while polyoxypropylene is abbreviated as POP.

In addition to aqueous, oil or gel diluents, other diluents which may be used in the compositions according to the invention comprise solvent systems containing ethyl alcohol, isopropyl alcohol, propylene glycol, polyethylene glycol, mixtures thereof or mixtures of one or more of the foregoing with water.

In any case, a pharmaceutically acceptable buffer should be present in order to create optimum pH conditions for both product stability and tolerance (pH range about 4 to about 8; preferably about 6.0 to 7.5). Suitable buffers include without limitation tris (tromethamine) buffer, phosphate buffer, etc. Preferably potassium phosphate NF is used to adjust the pH to 7.2.

Other excipients include chemical enhancers such as absorption promoters. These include fatty acids, bile acid salts and other surfactants, fusidic acid, lysophosphatides, cyclic peptide antibiotics, preservatives, carboxylic acids (ascorbic acid, amino acids), glycyrrhetinic acid, o-acylcarnitine. Preferred promoters are diisopropyladipate, POE(9) lauryl alcohol, sodium glycocholate and lysophosphatidyl-choline which proved to be particularly active.

Another excipient that may be present in a composition of this invention is a chelator, i.e. a substance that binds primarily di- or tri valent metallic ions (e.g. calcium) that might interfere with the stability or activity of the active ingredient. Chelators are known to those of skill in the art by referring to the recent edition of "Remington's Pharmaceutical Sciences." A preferred chelator is sodium ethylenediamine tetraacetic acid (sodium EDTA), USP.

Finally, the compositions of the invention preferably contain preservatives that ensure the microbiological stability of the active ingredient. Suitable preservatives include without limitation, methyl paraoxybenzoate (methyl paraben), propyl paraoxybenzoate (propyl paraben), sodium benzoate, benzyl alcohol, benzalkonium chloride and chlorobutanol.

The liquid ketorolac formulations, preferably in the form of solutions, may be administered in the form of drops or spray, using atomizers equipped with a mechanical valve and possibly including a propellant of a type commercially available, such as butane, N₂, Ar, C0₂, nitrous oxide, propane, dimethyl ether, chlorofluorocarbons (e.g., FREON) etc. Diluents (e.g. a solvent) suitable for spray administration are water, alcohol, glycol, glycerol, and propylene glycol, used alone or in a mixture of two or more. Preferably water is employed as the sole solvent.

The local anesthetics which can be used according to the invention are known per se and are listed, for example, in Remington's Pharmaceutical Sciences 1990, pp. 1048-1056. Local anesthetics are compounds which reversibly inhibit the excitability of sensory nerve endings or the neuronal conductivity for pain or other sensory stimuli in a limited region of the body without causing permanent harm (cf. J. L. McGuire (editor), Pharmaceuticals, volume 2, Wiley-VCH, Weinheim 2000, pp. 539, et seq.). Local anesthetics within the meaning of the present invention are preferably intended to mean substances which are listed in the Index Nominum 2000, International Drug Directory, Scientific Publishers Stuttgart 2000 with the therapeutic category "local anesthetic". Depending on the specific anesthetic used and the sensitivity of the patient, the anesthetic is present at a level of 4 to about 10% w/v.

Local anesthetics preferred according to the present invention are compounds of the formula (II) 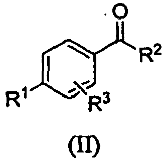 in which
R₁ represents H, NH₂, NH(C₁₋₆ alkyl), O-C₁₋₆- alkyl or CH₂OPh;
R² represents O-C₁₋₆-alkyl which may optionally have a radical from the group consisting of NH(C₁₋₆-alkyl), N(C₁₋₆-alkyl)₂ or a saturated 5- or six-membered heterocycle which contains at least one nitrogen atom and is linked via the latter, and optionally one or two further heteroatoms from the group consisting ofN, 0, S, and optionally carries one to three further C₁₋₆-alkyl radicals, or
R² represents (CH₂) ₁₋₆-Het, where Het represents a saturated 5- or six-membered heterocycle which contains at least one nitrogen atom and is linked via the latter, and optionally one or two further heteroatoms from the group consisting of N, 0, S, and optionally carries one to three further C₁₋₆-alkyl radicals; and
R³ represents H, halogen or O--C₁₋₆-alkyl.

Other useful compounds include those of the formula (III) 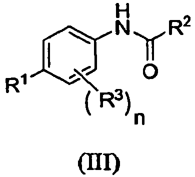 in which
R¹ represents H or OH;
R² represents C₁₋₆-alkyl-N(C₁₋₆-alkyl)₂ where the bridging alkyl chain may optionally carry one or more C₁₋₆-alkyl radicals, or represents a saturated 5- or six-membered heterocycle which contains at least one nitrogen atom and optionally one or two further heteroatoms from the group consisting of N, 0, S, and optionally carries one to three further C₁₋₆-alkyl radicals,
R³ represents C₁₆-alkyl, halogen or COOC₁₋₆-alkyl; and
n represents 1 or 2.

Other useful compounds are chosen from the group consisting of 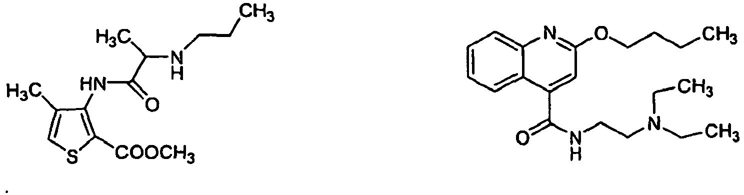 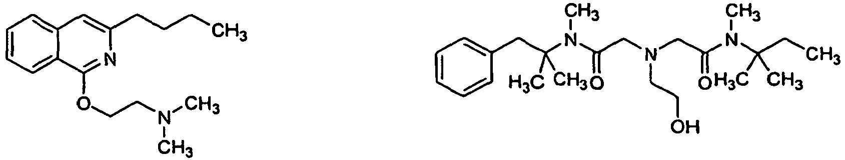 and polidocanol and benoxinate, and physiologically acceptable salts and/or hydrates thereof.

Particularly preferred local anesthetics according to the invention are those of the formula (II), above, in which
R¹ represents H, NH₂, NH-n-C₄H₉, O-n-C₃H₇, 0-n-C₄H₉ or CH₂OPh;
R² represents OC₂H₅, O-n-C₄H₉, O-(CH₂)₂N(C₂H₅)₂, O(CH₂)₂N(CH₃)₂, or a radical from the group consisting of 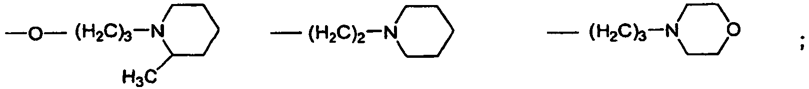 and
R³ represents H, Cl, O-n-C₃H₇ or O-n-C₄H₉.

Other preferred compounds are those of formula (III), above, in which
R¹ represents H or OH;
R² represents CH₂N(C₂H₅)₂, CHCH₃NH-n-C₃H₇, CH₂NH-n-C₄H₉ or a radical from the group consisting of 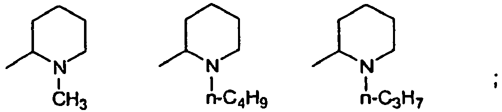 R³ represents CH₃, Cl or COOCH₃;
n represents 1 or 2;
and benoxinate and physiologically acceptable salts and/or hydrates thereof.

The local anesthetics that can be particularly preferably employed according to the invention are: benzocaine, butambene, piperocaine, piperocaine hydrochloride, procaine, procaine hydrochloride, chloroprocaine, chloroprocaine hydrochloride, oxybuprocaine, oxybuprocaine hydrochloride, proxymetacaine, proxymetacaine hydrochloride, tetracaine, tetracaine hydrochloride, nirvanin, lidocaine, lidocaine hydrochloride, prilocaine, prilocaine hydrochloride, mepivacaine, mepivacaine hydrochloride, bupivacaine, bupivacaine hydrochloride, ropivacaine, ropivacaine hydrochloride, etidocaine, etidocaine hydrochloride, butanilicaine, butanilicaine hydrochloride, articaine, articaine hydrochloride, cinchocaine, cinchocaine hydrochloride, oxetacaine, oxetacaine hydrochloride, propipocaine, propipocaine hydrochloride, dyclonine, dyclonine hydrochloride, pramocaine, pramocaine hydrochloride, fomocaine, fomocaine hydrochloride, quinisocaine, quinisocaine hydrochloride, benoxinate and polidocanol. These compounds are commercially available or can be prepared in a way known to the skilled person, for example as described in J. L. McGuire (editor), Pharmaceuticals, volume 2, Wiley-VCH 2000, pp. 539 et seq.

Local anesthetics that can preferably be used according to the invention are benzocaine, lidocaine , tetracaine, benoxinate, polidocanol or their pharmaceutically acceptable salts. Lidocaine, lidocaine hydrochloride, and lidocaine methanesulphonate are particularly preferred. The local anesthetic is present according to the invention at a level of 4 to about 10% w/v, preferably about 5-6% w/v. While 4-10% w/v is the general range, adjustments to narrow the range may be useful, e.g. 5-10%, 5-9%, 5-8%, 5-7%, and the like.

Illustrative formulations may contain the following ingredients and amounts (w/v) in addition to ketorolac, water, and the anesthetic.

| Ingredient | Broad Range (%) | | Preferred Range (%) | |
|---|---|---|---|---|
| Chelator (1) | 0.001 | - 1 | 0.01 | - 0.1 |
| Preservative (2) | 0 | - 2 | 0 | - 0.25 |
| Absorption promoter (3) | 0 | - 10 | 0 | - 10 |
| Gelling polymer (4) | 0 | - 5 | 0 | - 3 |
| Co-solvent (5) | 0 | - 99 | 0 | |

| | | | | |
|---|---|---|---|---|
| (1) *E.g*., sodium EDTA (2) *E.g*., methyl paraoxybenzoate or propyl paraoxybenzoate or mixtures thereof (3) *E.g*., sodium glycocholate (4) *E.g*., sodium CMC (5) *E.g*., glycerol | | | | |

It will be appreciated by those of ordinary skill that ingredients such as sodium CMC and polymers designated as CARBOPOL exist in many types differing in viscosity. Their amounts are to be adjusted accordingly. Different adjustments to each formulation may also be necessary including omission of some optional ingredients and addition of others. It is thus not possible to give an all-encompassing amount range for each ingredient, but the optimization of each preparation according to the invention is within the skill of the art.

Another alternative for the intranasal administration of the ketorolac-based compositions comprises a suspension of finely micronized ketorolac (generally from 1 to 200 micrometers, preferably from 5 to 100 micrometers) in a propellant or in an oily vehicle or in another vehicle in which the drug is not soluble. The vehicle is mixed or emulsified with the propellant. Vehicles suitable for this alternative are, for instance, vegetable and mineral oils and triglyceride mixtures. Appropriate surfactants, suspending agents and diluents suitable for use in pharmaceutics are added to these vehicles. Surfactants include, without limitation, sorbitan sesquioleate, sorbitan monooleate, sorbitan trioleate (amount: between about 0.25 and about 1 %); suspending agents include, without limitation, isopropyl myristate (amount: between about 0.5 and about 1%) and colloidal silica (amount: between about 0.1 and about 0.5%); and diluents include, without limitation, zinc stearate (about 0.6 to about 1%).

Compositions of the invention are administered to a patient in need thereof by contacting the patient's nasal passage, with an amount of the composition sufficient to result in absorption of ketorolac by the patient to reduce the pain and/or inflammation experienced by the patient. This is preferably carried out by spraying a solution, as described herein, into the nasal passage(s) of the patient from a vessel that is equipped with a device (e.g., an atomizer) for producing a spray (e.g. atomized particles). The device produces a mist or suspension of fine liquid particles that are inhaled by the patient into her or his nasal passage(s) from which it is rapidly absorbed into the bloodstream to effect its analgesic and anti-inflammatory action. Appropriate vessels and spray devices are available to one of skill in the art by referring to "Remington's Pharmaceutical Sciences." One source for such vessels is Ing. Erich Pfeiffer GmbH, Radolfzell, Germany. Another source is Valois, 50 avenue de l'Europe, 78164 MARLY-LE-ROI, France.

The following examples of formulations for the intranasal administration of ketorolac serve to illustrate the invention.

### EXAMPLE 1:

This example provides a description for making compositions for nasal administration in accordance with the invention. A solution was prepared in accordance with the proportions shown in Table 1. Lidocaine hydrochloride was added to the solution to give the compositions of the invention shown in Table 2.

**Table 1**

| | |
|---|---|
| Ketorolac tromethamine, USP | 15% |
| Sodium EDTA, NF | 0.02% |
| Potassium phosphate, NF | (q.s. to pH 7.2) |
| Water for Injection USP (q.s.) | 100 g |

**Table 2.**

| **Ingredients** | **I** | **II** | **III** |
|---|---|---|---|
| Ketorolac tromethamine, USP | 15% | 15% | 15% |
| Sodium EDTA, NF | 0.02% | 0.02% | 0.02% |
| Potassium phosphate, NF (q.s. to pH 7.2) | | | |
| Lidocaine hydrochloride | 4% | 5% | 6% |
| Water for Injection USP (q.s.) | 100 g | 100 g | 100 6 |

### EXAMPLE 2:

This example provides a description of a phase I clinical study (a double-blind, randomized 4-way crossover) carried out to determine, *i.a*., if the presence of lidocaine hydrochloride in nasally administered formulations of ketorolac has any adverse effect on the PK profile of ketorolac. The results show that the PK characteristics are improved in the preferred formulations (5-6% w/v) by the presence of the anesthetic lidocaine hydrochloride, and otherwise not adversely affected. In addition, the safety and tolerability data for the formulations were evaluated.

The clinical study included 16 healthy volunteers who were recruited for the study in which each volunteer received 4 nasally administered spray formulations, three of which are compositions that represent an aspect of the invention. There was a wash-out period of 3-7 days between each dose. The four aqueous spray compositions were prepared as follows:

| | KT(1) | L(2) | NaEDTA(3) | KPO₄(4) | Water(5) |
|---|---|---|---|---|---|
| A: | 15% | 0 | 0.02% | pH 7.2 | 100g |
| B: | 15% | 4% | 0.02% | pH 7.2 | 100g |
| C: | 15% | 5% | 0.02% | pH 7.2 | 100g |
| D: | 15% | 6% | 0.02% | pH 7.2 | 100g |

| | | | | | |
|---|---|---|---|---|---|
| (1) Ketorolac tromethamine, USP (2) Lidocaine hydrochloride, USP (3) Sodium EDTA, NF (4) Potassium phosphate, NF (5) Water for injection, USP | | | | | |

Formulation A is known and was included for comparative purposes. Formulations B-D are compositions of the invention with C and D being preferred.

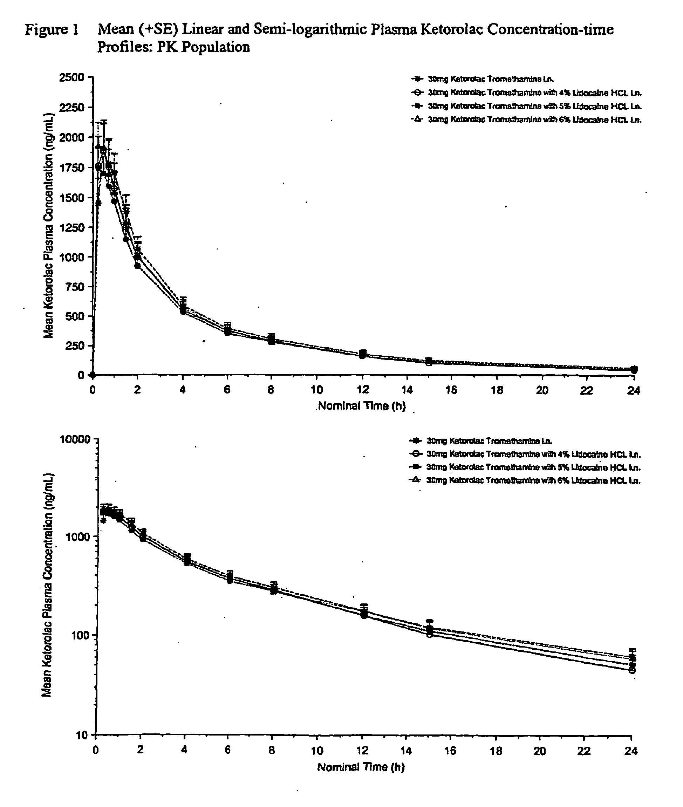

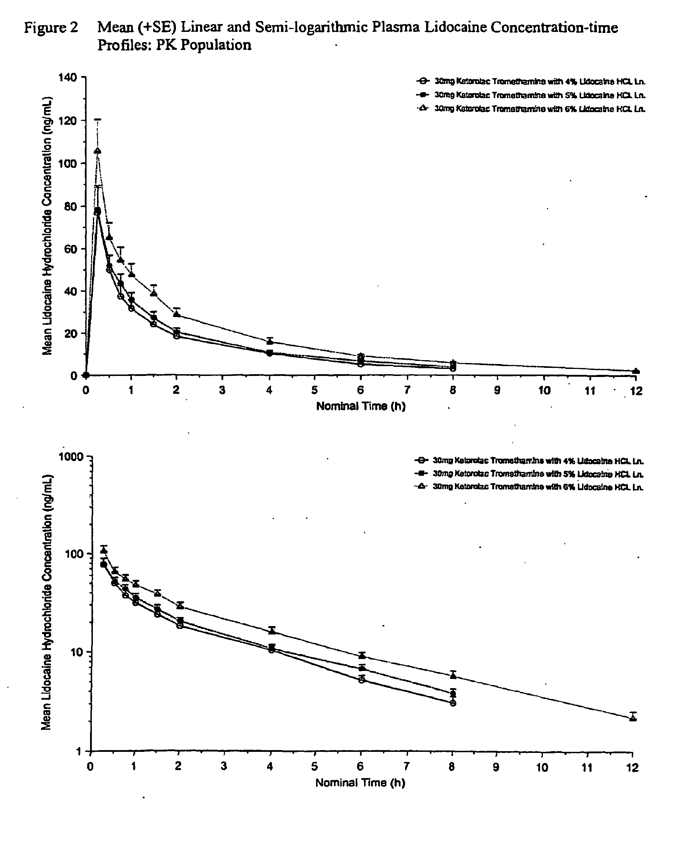

Blood was drawn from each participant as follows: Pre-dose (time(t) = 0 HR), 0.25, 0.50, 0.75, 1, 1.5, 2, 4, 6, 8, 12, 15, am 24 hours. The blood samples were analyzed for the levels of ketorolac and lidocaine to determine the ketorolac and lidocaine plasma concentrations in nanograms (ng) per milliliter (mL). These values were then combined to obtain the mean concentration time profiles in the participant population. The results for ketorolac are shown in Figure 1 (linear and semi-logarithmic), while the results for lidocaine are shown in Figure 2 (linear and semi-logarithmic). In each plot "SE" is the standard error. Each subject received a medical screening within 21 days of study drug administration in period 1. Subjects eligible to take part in the study took part in 4 periods. The subjects were admitted to the Unit on Day -1 of each period and remained resident until the morning of Day 2. A washout of 3-7 days occurred between each period. A post study medical was performed within 7 days of dosing on the last treatment period.

It is clear from Figures 1 and 2 that the PK for ketorolac is essentially unaffected by the presence of 4%, lidocaine, while the preferred formulation reduced the time to Cmax.

Each patient was evaluated by a clinician to determine the level of edema, erythema, ulceration, numbness, and stinging of the nasal passages. While the results tended to show that the subjects experienced reduced sensation of stinging, they were not conclusive due to difficulties in the evaluation process.

### EXAMPLE 3

This example describes a study for postoperative patients to compare the safety, tolerability, and pharmacokinetics (PK) of 3 formulations of intranasal (IN) ketorolac tromethamine.

The study is a double-blind, parallel study in postoperative patients. Subjects receive 1 of 3 formulations of IN ketorolac tromethamine 30 mg or placebo. Two of the formulations are preferred formulations according to this inventions. The study includes enrolling fifteen subjects in each group for a total of 60 subjects. Each formulation is administered to each subject IN (one spray into each nostril).

Postoperative patients suitable for the study include patients age 18 through 60 years, with body weight within 20% of ideal body weight as per the MetLife height and weight tables for men and women (version 1999).

Subjects are randomly assigned to receive either:
□ Treatment A (a composition known in the art) - single IN dose of 30 mg ketorolac tromethamine (one spray into each nostril of 100 µL of a 15% (pH 7.2) solution).
□ Treatment B (a preferred composition of this invention) - single IN dose of 30 mg ketorolac tromethamine with 5% lidocaine hydrochloride (one spray into each nostril of 100 µL of a 15% (pH 7.2) solution).
□ Treatment C (a preferred composition of this invention) - single IN dose of 30 mg ketorolac tromethamine with 6% lidocaine hydrochloride (one spray into each nostril of 100 µL of a 15% (pH 7.2) solution).
□ Treatment D - single IN dose of placebo (one spray into each nostril of 100 µL of vehicle solution).

### Procedures

| **Safety assessments** | |
|---|---|
| Clinical laboratory evaluations | Blood and urine samples for clinical laboratory tests are taken at screening. |
| | A urine pregnancy test is performed at screening for all women of childbearing potential. |
| Vital signs measurements and ECG recordings | Supine blood pressure and pulse are measured at screening and at the following timepoints during periods 1-4: predose (within 30 minutes of ketorolac administration), 2 h and 24 h postdose. |
| Tolerability assessments | Subjects are asked to complete a nasal irritancy questionnaire during all periods at the following timepoints: predose (within 15 minutes of ketorolac administration), 0-5 min, 15 min, 30 min, 1 h, and 24 h postdose. |
| Physical examination | A physical examination is performed at screening. |
| Nasal examination | A nasal examination is performed predose (within 30 minutes of ketorolac administration), 15 min, 30 min, 1 h, and 24 h postdose. |
| Adverse events | Subjects are queried about any adverse events they may have experienced at regular intervals throughout the study. |

### Data Analysis

Nasal irritation scores and adverse event data are tabulated by treatment group, physical findings and laboratory test results are listed by subject.

### APPENDIX OF PRODUCT NAMES AND EXAMPLES OF COMMERCIAL SOURCES

KETOROLAC TROMETHAMINE: Union Quimico Farmaceutico, S.A., Spain
HYDROXYPROPYLCELLULOSE (KLUCEL®) Dow Chemical Co, Midland MI USA
HYDROXYPROPYLMETHYLCELLULOSE (METHOCEL®) Dow Chem. Co, Midland MI
HYDROXYETHYLCELLULOSE (NATROSOL®) Hercules Inc, Wilmington DE USA
SODIUM CARBOXYMETHYLCELLULOSE (BLANOSE®) Hercules Inc, Wilmington DE
CARBOPOL®: BF Goodrich Chemical Co., Cleveland, OH, USA
POLYCARBOPHIL: BF Goodrich Chemical Co., Cleveland, OH, USA
GUM TRAGACANTH: Colony Ip. & Exp. Co., New York, NY, USA
GUM XANTHAN: Aldrich Chemie, Stanheim, Germany
SODIUM ALGINATE: Edward Mandell Co., Carmel, New York, USA
AGAR AGAR: Aldrich Chemie, Stanheim, Germany
POLOXAMER (LUTROL® f127): BASF Wyndotte Corp., Parsippany, NJ, USA
ETHYL ALCOHOL: Eastman Chemical Products Inc., Kingsport, TN, USA
ISOPROPYL ALCOHOL: Baker Chemical Co., New York, NY, USA
PROPYLENE GLYCOL: Dow Chemical Co., MIDLAND, MI, USA
POLYETHYLENE GLYCOL: BASF Wyndotte Corp., Parsippany, NJ, USA
DIISOPROPYLADIPATE: Croda, Goole, North Humerside, UK
SODIUM GLYCOCHOLATE: Sigma Chemical Company, St. Louis, MO, USA
LYSOPHOSPHATIDYLCHOLINE: American Lecithin, Long Island, NY, USA
METHYLPARAOXYBENZOATE (NIPAGIN): BDH Chemical Ltd, Poole, Dorset, UK
PROPYLPARAOXYBENZOATE: BDH Chemical Ltd, Poole, Dorset, UK
SODIUM BENZOATE: Pfizer Inc., New York, NY, USA.
BENZYL ALCOHOL: BDH Chemical Ltd, Poole Dorset, UK
BENZALCONIUM CHLORIDE: ION Pharmaceuticals, Covina, CA, USA
CHLORBUTANOL: Eastern Chemical Products, Smithtown, NY USA
SODIUM EDTA: Grace And Co., London, UK.
POE(9)LAURYL ALCOHOL: BASF Wyndotte Corp, Parsippany, NJ, USA
GLYCEROL: Dow Chemical Co., Midland, MI, USA
SODIUM CHLORIDE: Aldrich Chemie, Stanheim, Germany
GLUCOSE: Roquette Ltd, Tunbridge Wells, Kent, UK

## Claims

1. A composition for nasal administration to a patient that comprises a therapeutically effective amount of the compound 5-benzoyl-2,3-dihydro-1H-pyrrolizine-I-carboxylic acid, of the formula: 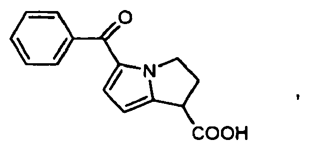 an optically active form thereof, a racemic mixture thereof, or a pharmaceutically acceptable salt thereof,
in combination with:
a pharmaceutically acceptable diluent and
4% w/v to 10% (w/v) of a local anesthetic.

2. A composition according to claim 1, wherein the compound is ketorolac tromethamine as a racemic mixture.

3. A composition according to claim 2, comprising 25 mg - 225 mg of said compound dissolved per mL, wherein the diluent is water and the composition is liquid and sprayable.

4. A composition according to claim 3, comprising 50-200 mg of said compound per mL.

5. A composition according to claim 2, wherein a chelator is present.

6. A composition according to claim 1, comprising 2.5-22.5% w/v of said compound dissolved in the diluent, which is water.

7. A composition according to claim 6, containing 15% w/v of said compound.

8. A composition according to claim 1, in a single-dose form suitable for administration to a single patient, wherein the composition is a sprayable liquid and the diluent is water.

9. A composition according to claim 1, in a multi-dose formulation, wherein the composition is a sprayable liquid and the diluent is water.

10. A composition according to claim 1, in the form of an aqueous solution.

11. A composition according to claim 1, wherein the anesthetic is lidocaine hydrochloride.

12. A composition according to claim 11, wherein the anesthetic is present at a level of 5% to 10% w/v, and the diluent is water, and the composition is a sprayable solution.

13. A composition according to claim 11, wherein the anesthetic is present at a level of 5% to 6% w/v.

14. A composition according to claim 1, wherein the pH is 6 to 7.5.

15. A composition according to claim 1, which is suitable for spraying into a human subject's nasal passage that comprises:
(a) water;
(b) the compound 5-benzoyl-2,3-dihydro-IH-pyrolizine-1-carboxylic acid, an optically active form thereof, a racemic mixture thereof, or a pharmaceutically acceptable salt thereof, present at a concentration ranging from 2.5% to 22.5% w/v;
(c) a local anesthetic, which is lidocaine, lidocaine hydrochloride, or lidocaine methanesulphonate, and which is present at a concentration of 4% to 10% w/v; and
(d) optionally other pharmaceutically acceptable excipients.

16. A composition according to claim 15, wherein the lidocaine, lidocaine hydrochloride, or lidocaine methanesulphonate is present at a concentration of 5% to 6% w/v.

17. A composition according to claim 15, wherein:
(a) water is the sole diluent;
(b) the compound is racemic ketorolac tromethamine dissolved at 2.5-22.5% w/v;
(c) the local anesthetic is lidocaine hydrochloride dissolved at 5-10% w/v; and
(d) the pH of the composition is adjusted to 7.2 and a chelator is present.

18. A composition according to claim 16 or 17, wherein:
the compound is present at a level of 15% w/v,
the local anesthetic is present at a level of 5-6% w/v,
potassium phosphate is the pH adjustor, and
the chelator is sodium EDTA.

19. A composition according to claim 1, wherein:
the compound is ketorolac tromethamine, which is present in the composition at 2.5-22.5% w/v;
the local anesthetic is lidocaine hydrochloride, which is present in the composition at 4-10% w/v; and
the composition is an aqueous, sprayable composition having a pH of 6-8.5.

20. A composition according to claim 19, wherein:
the compound is present at 15% w/v,
the local anesthetic is present at 5-6% w/v,
the pH is 7.2, and
sodium EDTA is present as a chelator.

21. A composition and vessel comprising a composition according to claim 1 contained in a vessel equipped with a device for spraying the composition into the nasal passage of a subject, wherein the composition is an aqueous solution.

22. A composition and vessel according to claim 21, wherein the vessel holds 50 to 2000 microliters (µL).

23. A composition according to any one of claims 1 to 22 for use in a method of treatment of the human or animal body by therapy.

24. A composition for use according to claim 23, for treatment of pain or inflammation.

25. A composition for use according to claim 24, for treatment of pain.

26. A composition for use according to claim 25, wherein the pain is pain in a subject that is the result of a trauma inflicted on the subject.

27. A composition for use according to claim 25, wherein the pain is pain in a subject that is the result of a medical operation performed on the subject.

28. A composition for use according to claim 25, wherein the pain is pathological pain.

29. A composition for use according to claim 25, wherein the pain is neuropathic pain.

30. A composition for use according to claim 25, wherein the pain is migraine or other headache pain.

31. Use of a composition according to any one of claims 1 to 22 in the manufacture of a medicament for the treatment of pain or inflammation.

32. Use according to claim 31 of a composition in the manufacture of a medicament for the treatment of pain.

33. Use according to claim 32 wherein the pain is pain in a subject that is the result of a trauma inflicted on the subject.

34. Use according to claim 32 wherein the pain is pain in a subject that is the result of a medical operation performed on the subject.

35. Use according to claim 32 wherein the pain is pathological pain.

36. Use according to claim 32 wherein the pain is neuropathic pain.

37. Use according to claim 32 wherein the pain is migraine or other headache pain.

## Patentansprüche

1. Zusammensetzung zur nasalen Verabreichung an einen Patienten, die eine therapeutisch wirksame Menge der Verbindung 5-Benzoyl-2,3-dihdro-1H-pyrrolizin-1-carbonsäure der Formel 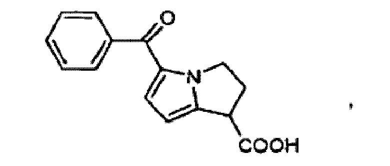 einer optisch aktiven Form davon, eines racemischen Gemisches davon oder eines pharmazeutisch annehmbaren Salz davon
in Kombination mit
einem pharmazeutisch annehmbaren Verdünner und
4 % (Gew./Vol.) bis 10 % (Gew./Vol.) eines Lokalanästhetikums umfasst.

2. Zusammensetzung nach Anspruch 1, wobei die Verbindung Ketorolactromethamin als racemisches Gemisch ist.

3. Zusammensetzung nach Anspruch 2, die 25 mg bis 225 mg der Verbindung pro ml gelöst umfasst, wobei das Verdünnungsmittel Wasser ist und die Zusammensetzung flüssig und sprühbar ist.

4. Zusammensetzung nach Anspruch 3, die 50 bis 200 mg der Verbindung pro ml umfasst.

5. Zusammensetzung nach Anspruch 2, wobei ein Chelatbildner vorhanden ist.

6. Zusammensetzung nach Anspruch 1, die 2,5 bis 22,5 % (Gew./Vol.) der Verbindung im Verdünnungsmittel, das Wasser ist, gelöst umfasst.

7. Zusammensetzung nach Anspruch 6, die 15 % (Gew./Vol.) der Verbindung enthält.

8. Zusammensetzung nach Anspruch 1 in einer Einzeldosisform, die zur Verabreichung an einen einzelnen Patienten geeignet ist, wobei die Zusammensetzung eine sprühbare Flüssigkeit und das Verdünnungsmittel Wasser ist.

9. Zusammensetzung nach Anspruch 1 in einer Mehrfachdosisform, wobei die Zusammensetzung eine sprühbare Flüssigkeit und das Verdünnungsmittel Wasser ist.

10. Zusammensetzung nach Anspruch 1 in Form einer wässrigen Lösung.

11. Zusammensetzung nach Anspruch 1, wobei das Anästhetikum Lidocainhydrochlorid ist.

12. Zusammensetzung nach Anspruch 11, wobei das Anästhetikum in einer Menge von 5 bis 10 % (Gew./Vol.) vorhanden ist, das Verdünnungsmittel Wasser ist und die Zusammensetzung eine sprühbare Lösung ist.

13. Zusammensetzung nach Anspruch 11, wobei das Anästhetikum in einer Menge von 5 bis 6 % (Gew./Vol.) vorhanden ist.

14. Zusammensetzung nach Anspruch 1, wobei der pH 6 bis 7,5 ist.

15. Zusammensetzung nach Anspruch 1, die zum Sprühen in den Nasengang eines menschlichen Individuums geeignet ist und Folgendes umfasst:
(a) Wasser,
(b) die Verbindung 5-Benzoyl-2,3-dihydro-1H-pyrrolizin-1-carbonsäure, eine optisch aktive Form davon, ein racemisches Gemisch davon oder ein pharmazeutisch annehmbares Salz davon, die/das in einer Konzentration von 2,5 bis 22,5 % (Gew./Vol.) vorhanden sind,
(c) ein Lokalanästhetikum, das Lidocain, Lidocainhydrochlorid oder Lidocainmethansulfonat ist und in einer Konzentration von 4 bis 10 % (Gew./Vol.) vorhanden ist, und
(d) gegebenenfalls andere pharmazeutisch annehmbare Exzipienten.

16. Zusammensetzung nach Anspruch 15, wobei das Lidocain, Lidocainhydrochlorid oder Lidocainmethansulfonat in einer Konzentration von 5 bis 6 % (Gew./Vol.) vorhanden ist.

17. Zusammensetzung nach Anspruch 15, wobei:
(a) Wasser das einzige Verdünnungsmittel ist;
(b) die Verbindung racemisches Ketorolactromethamin ist, das in einer Menge von 2,5 bis 22,5 % (Gew./Vol.) gelöst ist,
(c) das Lokalanästhetikum Lidocainhydrochlorid ist, das in einer Menge von 5 bis 10 % (Gew./Vol.) gelöst ist, und
(d) der pH der Zusammensetzung auf 7,2 eingestellt ist und ein Chelatbildner vorhanden ist.

18. Zusammensetzung nach Anspruch 16 oder 17, wobei:
die Verbindung in einer Menge von 15 % (Gew./Vol.) vorhanden ist,
das Lokalanästhetikum in einer Menge von 5-6 % (Gew./Vol.) vorhanden ist,
Kaliumphosphat das pH-Einstellmittel ist und
der Chelatbildner Natrium-EDTA ist.

19. Zusammensetzung nach Anspruch 1, wobei:
die Verbindung Ketorolactromethamin ist, das in der Zusammensetzung in einer Menge von 2,5 bis 22,5 % (Gew./Vol.) vorhanden ist,
das Lokalanästhetikum Lidocainhydrochlorid ist, das in der Zusammensetzung in einer Menge von 4 bis 10 % (Gew./Vol.) vorhanden ist, und
die Zusammensetzung eine wässrige, sprühbare Zusammensetzung mit einem pH von 6 bis 8,5 ist.

20. Zusammensetzung nach Anspruch 19, wobei:
die Verbindung in einer Menge von 15 % (Gew./Vol.) vorhanden ist,
das Lokalanästhetikum in einer Menge von 5 bis 6 % (Gew./Vol.) vorhanden ist,
der pH 7,2 beträgt und
Natrium-EDTA als Chelatbildner vorhanden ist.

21. Zusammensetzung nach Anspruch 1 und diese Zusammensetzung enthaltender Behälter, wobei die Zusammensetzung in einem Behälter enthalten ist, der mit einer Vorrichtung zum Sprühen der Zusammensetzung in den Nasengang eines Individuums ausgestattet ist, wobei die Zusammensetzung eine wässrige Lösung ist.

22. Zusammensetzung und Behälter nach Anspruch 21, wobei der Behälter 50 bis 2.000 Mikroliter (µl) enthält.

23. Zusammensetzung nach einem der Ansprüche 1 bis 22 zur Verwendung in einem Verfahren zur Behandlung eines menschlichen oder tierischen Körpers durch eine Therapie.

24. Zusammensetzung zur Verwendung nach Anspruch 23 zur Behandlung von Schmerz oder einer Entzündung.

25. Zusammensetzung zur Verwendung nach Anspruch 24 zur Behandlung von Schmerz.

26. Zusammensetzung zur Verwendung nach Anspruch 25, wobei der Schmerz Schmerz eines Individuums ist und das Ergebnis eines Traumas ist, welches das Individuum erlitten hat.

27. Zusammensetzung zur Verwendung nach Anspruch 25, wobei der Schmerz Schmerz eines Individuums ist und das Ergebnis eines chirurgischen Eingriffs ist, der am Individuum vorgenommen wurde.

28. Zusammensetzung zur Verwendung nach Anspruch 25, wobei der Schmerz pathologischer Schmerz ist.

29. Zusammensetzung zur Verwendung nach Anspruch 25, wobei der Schmerz neuropathischer Schmerz ist.

30. Zusammensetzung zur Verwendung nach Anspruch 25, wobei der Schmerz Migräne oder ein anderer Kopfschmerz ist.

31. Verwendung einer Zusammensetzung nach einem der Ansprüche 1 bis 22 zur Herstellung eines Medikaments zur Behandlung von Schmerz oder einer Entzündung.

32. Verwendung nach Anspruch 31 einer Zusammensetzung zur Herstellung eines Medikaments zur Behandlung von Schmerz.

33. Verwendung nach Anspruch 32, wobei der Schmerz Schmerz eines Individuums ist und das Ergebnis eines Traumas ist, welches das Individuum erlitten hat.

34. Verwendung nach Anspruch 32, wobei der Schmerz Schmerz eines Individuums ist und das Ergebnis eines chirurgischen Eingriffs ist, der am Individuum vorgenommen wurde.

35. Verwendung nach Anspruch 32, wobei der Schmerz pathologischer Schmerz ist.

36. Verwendung nach Anspruch 32, wobei der Schmerz neuropathischer Schmerz ist.

37. Verwendung nach Anspruch 32, wobei der Schmerz Migräne oder ein anderer Kopfschmerz ist.

## Revendications

1. Composition pour l'administration nasale à un patient qui comprend une quantité thérapeutiquement efficace du composé d'acide 5-benzoyl-2,3-dihydro-1H-pyrrolizine-1-carboxylique, de la formule: 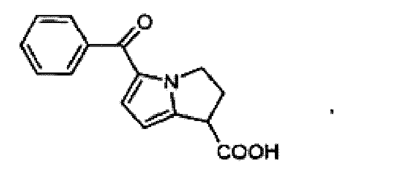 une forme optiquement active de celui-ci, un mélange racémique de celui-ci ou un sel pharmaceutiquement acceptable de celui-ci,
en combinaison avec:
un diluant pharmaceutiquement acceptable et
4% w/v à 10% (w/v) d'un anesthésique local.

2. Composition selon la revendication 1, où le composé est le kétorolac trométhamine comme mélange racémique.

3. Composition selon la revendication 2, comprenant 25 mg-225 mg dudit composé dissous par ml, où le diluant est de l'eau, et la composition est liquide ou peut être pulvérisée.

4. Composition selon la revendication 3, comprenant 50-200 mg dudit composé par ml.

5. Composition selon la revendication 2, dans laquelle un chélateur est présent.

6. Composition selon la revendication 1, comprenant 2,5-22,5% w/v dudit composé dissous dans le diluant, qui est de l'eau.

7. Composition selon la revendication 6, contenant 15% w/v dudit composé.

8. Composition selon la revendication 1, dans une forme à dose unique, apte à être administrée à un seul patient, où la composition est un liquide pouvant être pulvérisé, et le diluant est de l'eau.

9. Composition selon la revendication 1, dans une formulation multi-dose, où la composition est un liquide pouvant être pulvérisé, et le diluant est de l'eau.

10. Composition selon la revendication 1, sous la forme d'une solution aqueuse.

11. Composition selon la revendication 1, où l'anesthésique est le lidocaine hydrochlorure.

12. Composition selon la revendication 11, où l'anesthésique est présent au niveau de 5% à 10% w/v, et le diluant est de l'eau, et la composition est une solution apte à être pulvérisée.

13. Composition selon la revendication 11, dans laquelle l'anesthésique est présent à un niveau de 5% à 6% w/v.

14. Composition selon la revendication 1, où le pH est de 6 à 7,5.

15. Composition selon la revendication 1, qui convient pour être pulvérisée dans un passage nasal d'un sujet humain qui comprend:
(a) de l'eau;
(b) le composé d'acide 5-benzoyl-2,3-dihydro-1H-pyrolizine-1-carboxylique, une forme optiquement active de celui-ci, un mélange racémique de celui-ci ou un sel pharmaceutiquement acceptable de celui-ci, présent à une concentration de 2,5% à 22,5% w/v;
(c) un anesthésique local, qui est la lidocaine, le lidocaine hydrochlorure, ou le lidocaine méthanesulfonate, et qui est présent à une concentration de 4% à 10% w/v; et
(d) en option d'autres excipients pharmaceutiquement acceptables.

16. Composition selon la revendication 15, dans laquelle le lidocaine hydrochlorure ou lidocaine méthanesulfonate est présent à une concentration de 5% à 6% w/v.

17. Composition selon la revendication 15, où:
(a) l'eau est le diluant de sol;
(b) le composé est le kétorolac trométhamine dissout à 2,5-22,5% w/v;
(c) l'anesthésique local est le lidocaine hydrochlorure dissous à 5-10% w/v; et
(d) le pH de la composition est réglé à 7,2, et un chélateur est présent.

18. Composition selon la revendication 16 ou 17, où:
le composé est présent à un niveau de 15% w/v,
l'anesthésique local présent à un niveau de 5-6% w/v,
le potassium phosphate est l'ajusteur de pH, et
le chélateur est le sodium EDTA.

19. Composition selon la revendication 1, dans laquelle:
le composé est le kétorolac trométhamine, qui est présent dans la composition à 2,5-22,5% w/v;
l'anesthétique local est le lidocaine hydrochlorure, qui est présent dans la composition à 4-10% w/v; et
la composition est une composition aqueuse apte à être pulvérisée et ayant un pH de 6-8,5.

20. Composition selon la revendication 19, dans laquelle:
le composé est présent à 15% w/v,
l'anesthésique local est présent à 5-6% w/v,
le pH est de 7,2 et le sodium EDTA est présent comme chélateur.

21. Composition et récipient comprenant une composition selon la revendication 1, se trouvant dans un récipient équipé d'un dispositif pour pulvériser la composition dans le passage nasal d'un sujet, où la composition est une solution aqueuse.

22. Composition et récipient selon la revendication 21, où le récipient contient 50 à 2000 microlitres (µl).

23. Composition selon l'une quelconque 1 à 22 pour utilisation dans une méthode de traitement du corps humain ou animal par thérapie.

24. Composition pour utilisation selon la revendication 23, pour le traitement de la douleur ou de l'inflammation.

25. Composition pour utilisation selon la revendication 24, pour le traitement de la douleur.

26. Composition pour utilisation selon la revendication 25, où la douleur est une douleur chez un sujet qui est le résultat d'un trauma subi par le sujet.

27. Composition pour utilisation selon la revendication 25, où la douleur est une douleur chez un sujet qui est le résultat d'une opération médicale exécutée sur le sujet.

28. Composition pour utilisation selon la revendication 25, où la douleur est une douleur pathologique.

29. Composition pour utilisation selon la revendication 25, où la douleur est une douleur neurogène.

30. Composition pour utilisation selon la revendication 25, où la douleur est la migraine ou un autre mal de tête.

31. Utilisation d'une composition selon l'une quelconque des revendications 1 à 22, dans la fabrication d'un médicament pour le traitement de la douleur ou de l'inflammation.

32. Utilisation selon la revendication 31 d'une composition dans la fabrication d'un médicament pour le traitement de la douleur.

33. Utilisation selon la revendication 32, où la douleur est une douleur chez un sujet qui est le résultat d'un trauma subi par le sujet.

34. Utilisation selon la revendication 32, où la douleur est une douleur chez un sujet qui résulte d'une opération médicale exécutée sur le sujet.

35. Utilisation selon la revendication 32, où la douleur est une douleur pathologique.

36. Utilisation selon la revendication 32, où la douleur est une douleur neurogène.

37. Utilisation selon la revendication 32, où la douleur est la migraine ou un autre mal de tête.
